(19) Europäisches Patentamt
European Patent Office
Office européen des brevets

(11) EP 1 683 847 B1

(12) **EUROPEAN PATENT SPECIFICATION**

(45) Date of publication and mention of the grant of the patent:
**17.08.2011 Bulletin 2011/33**

(21) Application number: **04724172.4**

(22) Date of filing: **29.03.2004**

(51) Int Cl.:
***C09K 3/00*** *(2006.01)*
***C07D 487/22*** *(2006.01)*
***D06M 11/62*** *(2006.01)*
***D06M 19/00*** *(2006.01)*
***B68G 3/08*** *(2006.01)*

(86) International application number:
**PCT/JP2004/004445**

(87) International publication number:
**WO 2005/047414 (26.05.2005 Gazette 2005/21)**

# (54) ALLERGEN DECOMPOSER AND ANTIALLERGENIC FEATHER

ALLERGENZERSETZER UND ANTIALLERGENE FEDER

DECOMPOSEUR D'ALLERGENE ET PLUME ANTIALLERGENIQUE

(84) Designated Contracting States:
**AT BE BG CH CY CZ DE DK EE ES FI FR GB GR HU IE IT LI LU MC NL PL PT RO SE SI SK TR**

(30) Priority: **12.11.2003 JP 2003382565**
**17.11.2003 JP 2003387100**

(43) Date of publication of application:
**26.07.2006 Bulletin 2006/30**

(73) Proprietors:
- **Shinshu TLO Co. Ltd.**
  **Nagano-386-8567 (JP)**
- **Daiwabo Co., Ltd.**
  **Osaka-shi, Osaka 541-0056 (JP)**

(72) Inventors:
- **SHIRAI, Hirofusa**
  **Chiisagata-gun, Nagano 3860407 (JP)**
- **HIGAKI, Seigo**
  **Chuo-ku,**
  **Osaka-shi,**
  **Osaka 5410056 (JP)**
- **TSUIKI, Hisanaga**
  **Chuo-ku,**
  **Osaka-shi,**
  **Osaka 5410056 (JP)**
- **SUGIHARA, Yasuji**
  **Chuo-ku,**
  **Osaka-shi,**
  **Osaka 5410056 (JP)**

(74) Representative: **von Kreisler Selting Werner**
**Deichmannhaus am Dom**
**Bahnhofsvorplatz 1**
**50667 Köln (DE)**

(56) References cited:
**GB-A- 810 548**
**JP-A- 01 111 067**
**JP-A- 10 273 875**
**JP-A- 2001 214 367**

- **DATABASE WPI Week 198916 Derwent Publications Ltd., London, GB; AN 1989-120559 XP002452912 & JP 01 068578 A (DAIWA SPINNING CO LTD) 14 March 1989 (1989-03-14)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 683 847 B1

## Description

### Technical Field

This invention relates to the use of a chemical which contains a derivative of metal phthalocyanine as a mainly active ingredient and decomposes an allergen, a method to decompose the allergen by using the chemical, and antiallergenic feathers carrying a derivative of the metal phthalocyanine.

### Background Art

As for a symptom of allergy, in case an allergen invades a human body through skin, respiratory organs and digestive organs, an antigen-antibody reaction which humans have by nature induces an isolation in the body of a chemical transmitter like histamine and leukotriene etc., or forming active oxygen, and fever, rash, itching, vomiting and nasal inflammation etc. appear as a secondary symptom. These symptoms of allergy appear depending on a combination of a sort of an allergen and a constitution which each human has.

The allergen is contained as ingredients of a tick, its carcass and discharges, pollen of cedar, ragweed and orchard grass etc., bacterium, mold, egg, milk, seafood, soybean, insect, hair of animals, and dandruff of animals or human, and most of allergens are protein. With conditions that there is no medicine to effect on the symptom of allergy, to prevent appearance of the symptom of allergy, it is important for humans with a symptom of allergy to isolate an allergen. However, as many allergens are in the nature and accompanying with things which are necessary for human living, it is so difficult to remove them from living area thoroughly, and it is impossible essentially.

As a method to remove appearing allergens, Japanese Patent Provisional Publication No. 2000-5531 shows an antiallergenic filter. Tea polyphenol as extractive ingredient of tea is stuck to the filter as an inactivator of allergy. Japanese Patent Provisional Publication No. 2001-214367 shows antiallergenic fibers containing zirconium salt which reacts to the allergen and makes it inactive such as cotton, hemp, wool, silk, rayon, nylon, polyester and acrylic resin etc., and a fiber product thereof such as beddings, masks and curtains.

And as a method to prevent the tick from the fiber product, treatment in a tick repellent is well known. A small amount of the tick repellent may effect on just avoiding, a large amount of the tick repellent will be required to kill the tick, so there is a limit to the amount thereof in the point of safety on humans. Moreover, no inhibiting effect of the tick repellent is confirmed on a symptom of allergy which appears by the allergens derived from the tick like tick carcass or discharges.

The feather products of waterfowls are used generally as a material of beddings and clothes because of their superiority on keeping warm, keeping humidity and breathability. The amount of an allergen derived from the tick is liable to increase remarkably in the feather product because the tick eats feathers as animal protein, the product moderately adsorbs moisture caused by human's sweat etc., the product is heated moderately by human's temperature, and the product makes an environment for tick's increasing. Antiallergenic feathers, which is not treated with the tick repellent, is safety and has an effect on adsorbing the allergen derived from tick, is not known.

Whereas, Japanese Patent Provisional Publication Nos. 56-63355 and 61-258806 show a deodorizer which contains metal phthalocyanine as an active ingredient. Japanese Patent Provisional Publication No. 61-258806 describes that a derivative of metal phthalocyanine has an effect as a deodorizer by reacting as a catalyst on an oxidation-reduction reaction.

### Disclosure of Invention

As the results of our long researches on the enzyme-like catalyst reaction of metal phthalocyanine, we have eventually found that the protein is denatured by ability of adsorbing of the metal phthalocyanine and reacting as a catalyst on an oxidation-reduction reaction thereof. Considering that it is so difficult to remove the generation of the allergen thoroughly, it is the first object of the present invention to provide an allergen decomposer by the characteristic of metal phthalocyanine and the method for decomposing allergens by the decomposer. And it is the second object of the present invention to provide antiallergen feathers and the composition or the feather product containing it which do not require a tick repellent and have an effect on an adsorption to tick and the allergens derived from tick like tick carcass or discharges etc.

An allergen decomposer of the present invention developed for accomplishing the foregoing first object comprises metal phthalocyanine phthalocyanine derivative is a compound represented by the following formula (II) or phthalocyanate thereof as an active ingredient

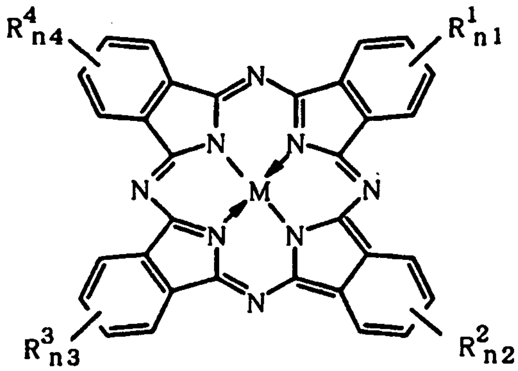

· · · (II)

(in the formula (II), M is same as the formula (I); $R^1{}_{n1}$, $R^2{}_{n2}$, $R^3{}_{n3}$ and $R^4{}_{n4}$ are substituents that $R^1$, $R^2$, $R^3$, $R^4$ are same or different to each other and are at least COOH group or $SO_3H$ group, n1, n2, n3, n4 are same or different to each other and are 0 to 4, and are numbers of substituents that satisfy $1 \leq n1+n2+n3+n4 \leq 8$).

In the allergen decomposer, the metal phthalocyanine derivative is metal phthalocyanine dicarboxylic acid, metal phthalocyanine tetracarboxylic acid, metal phthalocyanine octacarboxylic acid, metal phthalocyanine disulfonic acid, metal phthalocyanine tetrasulfonic acid, metal phthalocyanine octasulfonic acid, or carboxylate or sulfonate thereof.

The metal phthalocyanine dicarboxylic acid is represented by the following formula (III).

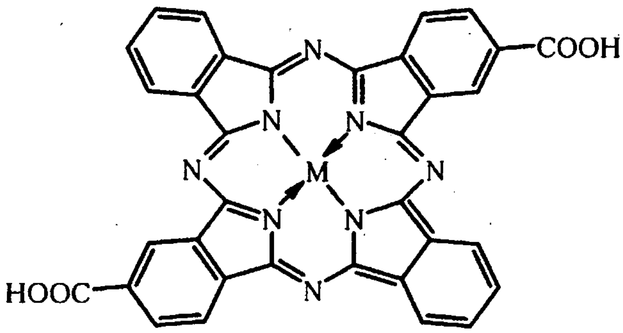

· · · (III)

The metal phthalocyanine tetracarboxylic acid is represented by the following formula (IV).

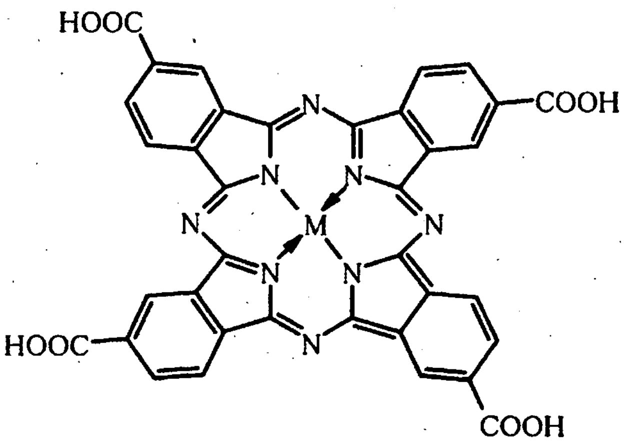

· · · (IV)

The metal phthalocyanine octacarboxylic acid is represented by the following formula (V).

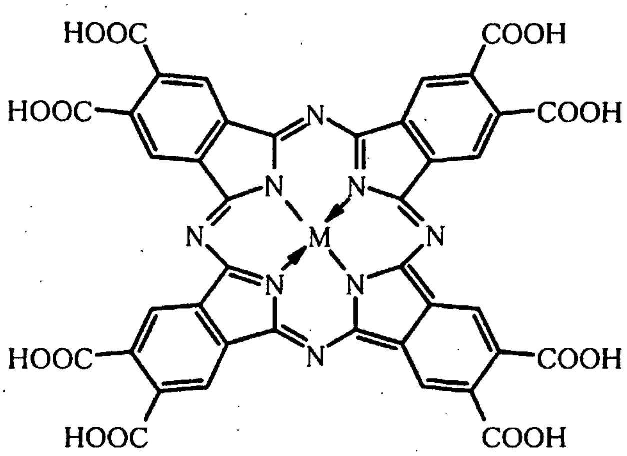

· · · (V)

The metal phthalocyanine disulfonic acid is represented by the following formula (VI).

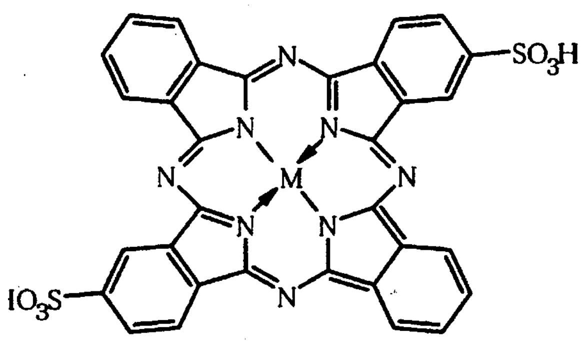

· · · (V I)

The metal phthalocyanine tetrasulfonic acid is represented by the following formula (VII).

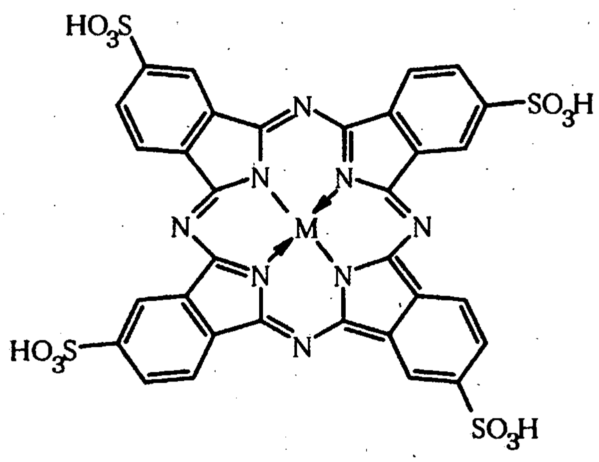

· · · (V I I)

The metal phthalocyanine octasulfonic acid is represented by the following formula (VIII).

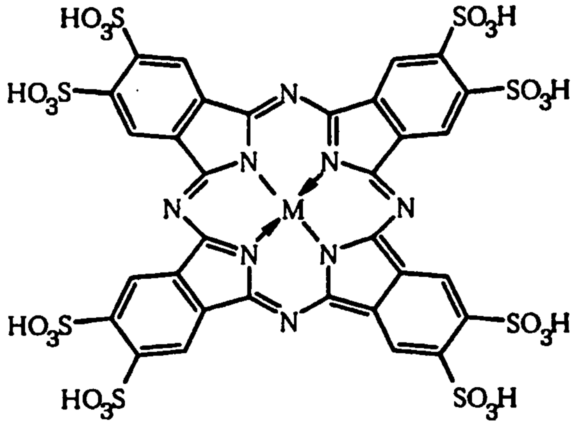

• • • (V I I I)

For the allergen decomposer, the allergen is an allergen derived from a protein.

In the allergen decomposer, the metal phthalocyanine derivative is carried or mixed to the carrier.

A method for decomposing an allergen of the present invention developed for accomplishing the foregoing first object is that the allergen decomposer comprising the metal phthalocyanine derivative as an active ingredient presented by the formula (II) is placed into a living environment.

The derivative of the metal phthalocyanine as the active ingredient of the allergen decomposer on the present invention coordinates the allergen protein on its central metal, and cuts the peptide bindings to be a lower molecular or change the molecular structures by the oxidation with oxygen in the air. As the derivative of the metal phthalocyanine not only adsorbs the allergen but also acts as the catalyst in decomposing, its action is permanent. When the allergen invades a human body, it binds peculiarly to an antibody in human by the molecular structure thereof and appears the symptom of allergy. But in case the allergen is lower molecule or molecular structure thereof changes, it does not bind to the antibody.

As most of the allergens in the living environment are decomposed by this decomposer, in case a little amount of the allergens invade human bodies with an allergenic constitution, the concentration thereof doesn't reach to threshold for appearing the symptom of allergy. As a result, if the allergen decomposer of the present invention is in the living environment, human with an allergenic constitution can live daily life without appearing a symptom of allergy.

Antiallergenic feathers of the present invention developed for accomplishing the foregoing second object carries metal phthalocyanine derivative described previously to feathers thereof.

In the metal phthalocyanine derivative represented by the formula (II) used above, it is preferable that $R^1$, $R^2$, $R^3$, $R^4$ are $SO_3H$ group and n1, n2, n3, n4 are 1. And M in the formula (II) is preferably Co or Fe.

The metal phthalocyanine derivative of the antiallergenic feathers is sodium salt or copper(II) salt of the compound represented by the formula (II).

The amount of the metal phthalocyanine derivative in the antiallergenic feathers is 0.1 mass% or more and 10 mass% or less to weight of the feathers.

The composition of the present invention is also characterized by containing the antiallergenic feathers described previously.

The feather product of the present invention is also characterized by containing the antiallergenic feathers described previously.

These antiallergenic feathers, the composition and feather product containing thereof are not treated with a tick repellent, and have an effect on adsorption to not only tick but also the allergens derived from tick like tick carcass or discharges etc. Therefore, the allergen derived from tick can be removed.

Brief Description of Drawings

Fig. 1 shows a photograph of the two-dimensional electrophoresis gel using the test solutions on preparing example 1 and comparative preparing example 1.

Fig. 2 shows a photograph of the two-dimensional electrophoresis gel using the test solutions on preparing example 2 and comparative preparing example 2.

Fig. 3 shows a photograph of the two-dimensional electrophoresis gel using the test solutions on preparing example 3 and comparative preparing example 3.

Fig. 4 shows a photograph of the two-dimensional electrophoresis gel using the test solutions on preparing example 4 and comparative preparing example 4.

Best Mode for Carrying Out the Invention

Hereunder, the embodiments of the present invention are explained in detail. However, the present invention is not limited to the examples as follows.

First, the allergen decomposer and the method for decomposing the allergens applicable for the present invention will be more clearly understood.

The allergen decomposer of the present invention contains as an active ingredient the derivative of the metal phthalocyanine described as the formula (II), and M is selected among the metals described previously, particularly iron, cobalt, or copper are desirable. The most desirable is iron phthalocyanine tetracarboxylic acid.

The compounds of metal phthalocyanine (chemical formula II) or phthalocyanate thereof can be available from the market, or produced in a well-known method. For example, they can be produced in the method described in "phthalocyaninechemistry and function-" (written by Hirofusa Shirai and Nagao Kobayashi, published by Industrial Publishing & Consulting, Inc., published in February 28, 1997). For example, iron phthalocyanine tetracarboxylic acid can be prepared by the method as follows. Nitrobenzene is added to trimellitic anhydride, urea, ammonium molybdate and ferric chloride anhydride, and then stirred, refluxed under heating and the precipitation is prepared. Alkali is added to the prepared precipitation, then the precipitation is hydrolyzed, and acid is added thereto for acidifying, and iron phthalocyanine tetracarboxylic acid can be prepared. Cobalt phthalocyanine octacarboxylic acid can be produced in the same method by using pyromellitic anhydride instead of trimellitic anhydride as the material of iron phthalocyanine tetracarboxylic acid described above, and cobalt(II) chloride instead of ferric chloride anhydride.

The derivative of the metal phthalocyanine is desirable to be the allergen decomposer by carrying or mixed with a carrier of organic matters or inorganic matters. As the carrier of organic matters, fibers are more desirable. Since fibers are bulky and have a large surface area, metal phthalocyanine or the derivative thereof can touch the allergens in the air efficiently.

As the materials of fibers, all of the natural fibers, recycled fibers, partly-synthetic fibers and synthetic fibers can be used. For example, cellulose fibers (cotton, hemp, rayon etc.), protein fibers (wool, silk etc.), polyamide fibers, polyester fibers, polyacryl fibers, poval fibers, polyvinyl chloride fibers, polyvinylidene chloride fibers, polyolefin fibers and polyurethane fibers etc. Particularly cellulose fiber, especially cotton has a good condition to demonstrate the enzyme-like function as a medium absorbing water because of its ability of suction.

The fibers carrying the allergen decomposer decompose, which are used as clothes or beddings, isolate the allergens from a body with allergic constitution and decompose the allergens. The fibers, which are used as curtains, cut off the allergens came from outdoors and decompose the allergens. The fibers, which are used as wallpapers or carpets, decompose the floating or laying allergens. The fibers, which are used as air filters, decompose the passing allergens.

The allergen decomposer and the method of decomposing of the present invention will be more clearly understood with reference to the following example 1.

(Example 1)

In order to prove the validity of the allergen decomposer of the present invention, drug efficacy of iron phthalocyanine tetracarboxylic acid as one of the derivatives of metal phthalocyanine was confirmed by *in vitro* experiments. And safety pharmacology thereof was done by *in vivo* animal experiments and other experiments.

(Confirmation of drug efficacy)

Tick antigen Derf II (available from ASAHI BREWERIES, LTD.) was chosen between the allergens, and we compared electrophoresis of the tick allergen containing iron phthalocyanine tetracarboxylic acid with that of the allergen only.

1. Preparation of solutions

Iron phthalocyanine tetracarboxylic acid potassium salt (Fe-Pc-COOK) aqueous solution and Derf II solution, which appropriate concentrations (w/v%) were mentioned in Table 4, were mixed with swelling stock solution containing

IPG (Immobilized pH Gradient) buffer (8mol/L Urea, 2w/v% CHAPS, 2% IPG Buffer, appropriate amount of Bromophenol blue, and distilled water) to prepare experimental solutions of preparing examples 1-4 in Table 4.

Whereas Derf II solution was mixed with swelling stock solution containing IPG buffer to prepare experimental solutions of comparative preparing examples 1-4 in Table 4.

2. One-dimensional electrophoresis

One-dimensional electrophoresis gel films (Strip) of Strip pH4-7 and Strip pH 3-10.5 were prepared, soaked in each experimental solution containing appropriate amount of silicon oil for preventing dry, and treated for 10 hours. After being pulled up and washed, each Strip was subjected to one-dimensional electrophoresis containing silicon oil for preventing dry for 16 hours.

One-dimensional electrophoresis programs of Strip pH4-7 were shown as Table 1.

Table 1

| Phase | Voltage (V) | Electric Current (mA) | W | Time (hr.) | V·hr. |
|---|---|---|---|---|---|
| 1 | 300 | 1 | 5 | 0:01 | 1 |
| 2 | 300 | 1 | 5 | 4:00 | 1200 |
| 3 | 3500 | 1 | 5 | 5:00 | 9500 |
| 4 | 3500 | 1 | 5 | 7:00 | 24500 |
| Total | | | | 16:01 | 35201 |

One-dimensional electrophoresis programs of Strip pH 3-10.5 were shown as Table 2.

Table 2

| Phase | Voltage (V) | Electric Current (mA) | W | Time (hr.) | V.hr. |
|---|---|---|---|---|---|
| 1 | 300 | 1 | 5 | 0:01 | 1 |
| 2 | 300 | 1 | 5 | 3:00 | 900 |
| 3 | 2000 | 1 | 5 | 5:00 | 5750 |
| 4 | 2000 | 1 | 5 | 8:00 | 16000 |
| Total | | | | 16:01 | 22651 |

3. Two-dimensional electrophoresis (the same method for each Strip of one-dimensional electrophoresis)

After one-dimensional electrophoresis, Strip was pulled up, washed, soaked in 10mL of SDS(sodium dodecyl sulfate) equilibrium buffer (1.5mol/L pH8.8 Tris-Cl 50mmol/L, 6mol/L Urea, 87v/v% Glycerol 30v/v%, 2v/v% SDS, appropriate amount of Bromophenol blue, and distilled water) containing 100mg/10mL DTT (dithiothreitol), and treated for 10 minutes. And then, it was pulled up, soaked in 10mL of SDS equilibrium buffer containing 250mg/10mL iodoacetamide, and treated for 10 minutes. After being pulled up, washed and wiped, each Strip was subjected to two-dimensional electrophoresis with filter paper, electrode potential gel, and molecular weight markers for 1 hour and 40 minutes.

Two-dimensional electrophoresis programs were shown as Table 3.

Table 3

| Phase | Voltage (V) | Electric Current (mA) | W | Time (min.) |
|---|---|---|---|---|
| 1 | 600 | 20 | 30 | 25-30 |
| 2 | 600 | 50 | 30 | 5 |
| 3 | 600 | 50 | 30 | 70 |

4. Stain and photograph

Gels were stained with Silver Staining Kit, Protein (available from Amersham Biosciences).

Two-dimensional electrophoresis gels were soaked in fixing solution (100mL of ethanol, 25mL of acetic acid, and distilled water for diluting to 250mL), and treated for 30 minutes. After that, they were soaked in sensitizing solution (75mL of ethanol, 1.25mL of 25w/v% glutaraldehyde, 10mL of 5w/v% sodium thiosulfate, 17g sodium acetate, and distilled water for diluting to 250mL), and treated for 30 minutes. After being soaked to wash in 250mL of distilled water for 5 minutes three times, they were soaked in silver reaction solution (25mL of 2.5w/v% silver acetate, 0.1mL of 37w/v% formaldehyde, and distilled water for diluting to 250mL), and treated for 20 minutes. After being soaked to wash in 250mL of distilled water for 30 minutes twice, they were soaked in developing solution (6.25g of sodium carbonate, 0.05mL of 37w/v% formaldehyde, and distilled water for diluting to 250mL), and treated for 2-5 minutes. After being soaked in stop solution (3.65g of EDTA-$Na_2 \cdot 2H_2O$ and distilled water for diluting to 250mL) for 10 minutes, they were soaked to wash in 250mL of distilled water for 5 minutes three times.

These two-dimensional electrophoresis gels stained by silver were photographed with Printgraph-1 electrophoresis photograph device (available from ATTO Corporation).

Each concentration of Fe-Pc-COOK, pH of one-dimensional electrophoresis Strip, concentration gradient of polyacrylamide in two-dimensional electrophoresis gel, and Figure No. indicating photograph of the two-dimensional electrophoresis gel were shown as Table 4.

Table 4

| Example | Solutions | pH of Strip | Gradient | Fig. No. |
|---|---|---|---|---|
| Preparing Example 1 | 20μL of Derf II sol. and 20μL of | 3-10 | 8-18 | Fig.1 A |
| Comparative Preparing | 20μL of Derf II sol. | 3-10 | 8-18 | Fig.1 B |
| Preparing Example 2 | 20μL of Derf II sol. and 20μL of | 4-7 | 8-18 | Fig.2 A |
| Comparative Preparing | 20μL of Derf II sol. | 4-7 | 8-18 | Fig.2 B |
| Preparing Example 3 | 30μL of Derf II sol. and 30μL of | 4-7 | 8-18 | Fig-3 A |
| Comparative Preparing | 30μL of Derf II sol. | 4-7 | 8-18 | Fig.3 B |
| Preparing Example 4 | 70μL of Derf II sol. and 30μL of | 4-7 | 8-18 | Fig.4 A |
| Comparative Preparing | 70μL of Derf II sol. | 4-7 | 8-18 | Fig.4 B |

The positions of the molecular weight markers are shown on the center of Figs. 1-4, which indicates appropriate molecular weight. In Figs. 1-3, each spot of the molecular weight markers indicates 14.4, 20.1, 30, 45, 66, 97 K Da (Kiro Dalton) from the bottom. In Fig. 4, each spot of the molecular weight markers indicates 3.5, 6.5, 14.3, 20.1, 30, 45 K Da from the bottom. Each abscissa of A (on the left) and B (on the right) in each Fig. shows pH of one-dimensional electrophoresis Strip.

A in each Fig. shows the tick allergen containing iron phthalocyanine tetracarboxylic acid potassium salt, and B in each Fig. shows the tick allergen only. If iron phthalocyanine tetracarboxylic acid potassium salt does not decompose the tick allergen, the spots in A and B should be the same. Disappearance of the spots between pH 5-6 in A reveals that iron phthalocyanine tetracarboxylic acid potassium salt may have an effect on the tick allergen.

(Confirmation of safety pharmacology)

In order to prove the safety of the allergen decomposer of the invention, experiments on iron phthalocyanine tetracarboxylic acid were performed as follows.

1. Irritation experiment on rabbit skin (for once)

Knit containing 1w/w% iron phthalocyanine tetracarboxylic acid was used as functional fiber of the allergen decomposer.

On the preceding day, 6 male rabbits at the age of 17 weeks (Japanese White, Kb1:JW(SPF)) with healthiness and unwounded skin were prepared, and clipped on the back to locate into 4 spots (2.5 X 2.5 cm / spot) for sticking. 2 spots were used as normal skin, and the other 2 spots were used as injured skin after lightly shaving and stripping with cellophane tape. Each one spot of the normal and injured skins was stuck on with the functional fiber moistened with

injection water, covered with gauze, and taped to fix them. The other 2 spots were covered with gauze and taped. Furthermore, these 4 spots were covered with a fabric cover and a cylindrical net bandage. After 24 hours, the functional fiber, the gauze and the tape were removed. These 4 spots were wiped with lukewarm water.

These 4 spots were observed macroscopically before sticking the functional fiber and at 1, 24, 48 and 72 hours after removing thereof. Erythema (scabbing) and edema were scored following Draize scoring criteria (Appendix 3) respectively. Classifying the normal and injured skins, the mean and standard deviation of erythema (scabbing) score, edema score and total score (TS) were calculated as of observations. Primary irritation indexes (mean of TS: PII) using of each score thereof at 24, 48 and 72 hours after removing were calculated following ISO10993-10 scoring criteria (Appendix 3), and irritation strength of the functional fiber was evaluated by the indexes.

No irritation reaction was confirmed on every spot of the normal and injured skins stuck on with the functional fiber throughout observations. PII of the functional fiber were 0.0 on every spot of the normal and injured skins. The irritation strength of the functional fiber was evaluated as "negligible", and safety of the functional fiber on the skin was confirmed.

2. Irritation experiment on rabbit skin (repeatedly)

The same functional fiber of the allergen decomposer and injured skin of rabbit were prepared. Each one spot of the normal and injured skins was stuck on with the functional fiber moistened with injection water, covered with gauze and taped to fix them. The other each spot was covered with gauze and taped. Furthermore, these spots were covered with a fabric cover and a cylindrical net bandage. After 23 hours, the functional fiber, the gauze and the tape were removed. These 4 spots were wiped with lukewarm water. These 4 spots were observed macroscopically at 1 hour after removing and before sticking the functional fiber everyday. The procedures were repeated for 21 days. Erythema (scabbing) and edema were scored following Draize scoring criteria respectively. Classifying the normal and injured skins, the mean and standard deviation of erythema (scabbing) score, edema score and total score (TS) were calculated as of observations. After 21 days, all rabbits were bled to death under pentbarbital sodium anethesia. Each skin of 4 spots was fixed in 10% neutral formalin buffer, stained in HE and examined histopathologically.

No irritation reaction was confirmed on every spot of the normal and injured skins stuck on with the functional fiber throughout observations. No histopathological change was confirmed. These results reveal that safety of the functional fiber on skin was confirmed.

3. Irritation experiment on rabbit eye mucosa

Male rabbits at the age of 10 weeks (Japanese White, Kb1:JW(SPF)) were prepared for a washed group (n=3) and a non-washed group (n=3). After pre-bred for 7 days, the rabbits were confirmed normality about changes of body weight and health condition. On the preceding day, they were confirmed about normality of eyes macroscopically and no injury of cornea by fluoresceine staining (fluor-paper, Lot No. 3990849 :available from Wyeth-Ayerest Laboratories, USA) using slitlamp (type SL-5 :available from Kowa Company, Ltd.) to prepare for experiment.

100mg of iron phthalocyanine tetracarboxylic acid powder, which was screened through a mesh having 300$\mu$m of aperture once, was put into right conjunctiva of each rabbit, and upper and lower eyelids were gently kept closed for about one second. In the washed group, to remove powder, the conjunctiva was washed with 230-330mL of lukewarm water at 30 seconds after putting to remove the powder. The left conjunctiva of each rabbit wasn't treated in both groups. The cornea, iris and the conjunctiva in both groups were compared with non-treated eye as reference at 1, 24, 48 and 72 hours after putting. Irritation reaction was observed at 72 hours after putting in the non-washed group, the observation was continued until 21$^{st}$ day. The observation results were scored following Draize scoring criteria (Appendix 3), and the irritation was evaluated following Kay and Calandra classification (Appendix 4).

In the non-washed group, the irritation reaction was expressed clearly in all the rabbit eyes. Corneal opacity and rough, congested iris, red conjunctiva, edema, secretion, and so on were observed. Furthermore, coloring to green of the cornea and the iris by the powder was observed. These reactions were expressed the most in 24 hours after putting. After that gradual recover was observed, the reactions of the cornea and the iris disappeared by 72 hours after putting, and that of the conjunctiva by 10$^{th}$ day. Just colored iris was observed continually by 21$^{st}$ day. The irritation on the rabbit eye mucosa was evaluated as "moderately irritating".

In the washed group, slight red conjunctiva of all the eyes was observed at 1 hour after putting, which disappeared by 24 hours after putting. No change was observed in the cornea and the iris. The irritation on the rabbit eye mucosa was evaluated as "practically nonirritating". It was confirmed that iron phthalocyanine tetracarboxylic acid has "moderately irritating ($M_3$)" stimulus to the rabbit eye mucosa; however, the irritation reactions are recoverable and relieved to "practically nonirritating (P)" by being washed.

4. Intravenous injection of an extract to rats

The functional fiber was soaked in saline (844mg of sodium chloride, 1200mg of potassium chloride, 146mg of calcium chloride, 52mg of magnesium chloride, 342mg of dipotassium phosphate, and 1000mL of purified water). The ratio of fiber to saline was 0.2g/1mL. An extract was prepared by being autoclaved at the temperature of 121±2 degrees centigrade for 1 hour, cooled down to 20-30 degrees centigrade, kept at room temperature and used within 24 hours.
We studied on toxicity of the repeated (for 28 days) intravenous injection of the extract of the functional fiber in saline (20mL/kg, equivalent to 4g fiber/kg) to 26 male and female rats (Crj:CD(SD)IGS(SPF)) at the age of 6 weeks. In a comparative group, rats were injected of saline. As a result, no rat died during the experiments.
The researches on health condition, change of body weight, change of amount of food, pathological anatomy and histopathology didn't reveal the changes suggesting the influence in injection of the extract thereof. The some researches on particulars of urine, blood, bloody component, and organ weight showed significant differences between a group of the injection of the extract of the functional fiber and the comparative group. It is appeared that every significant difference was accidental and irrelevant to toxicity, because the differences were hard to discriminate from variable background data, and could not be estimated based on any other researches of the groups that were the same.
In conclusion, the extract of the functional fiber in saline is thought to be very low toxicity, and innocuous dose under this experiment in the male and female rats is suggested over 20mL/kg of the extract that is equivalent to 4g fiber/kg.

5. Toxicity experiment by oral injection of the extract to rats (for once)

26 male rats and 26 female rats (Crj:CD(SD)IGS(SPF)) at the age of 5 weeks were housed for 1 week before experiment, and all rats were considered to be available and used to the experiment.
They were divided into each group according to their body weights measured before housing by a continuous stratified sampling method. Extra rats caused by the method were excluded from the experiment. They were at the age of 6 weeks, 178-197g weigh (male), 122-142g weigh (female).
The functional fiber was soaked in artificial saliva (844mg of sodium chloride, 1200mg of potassium chloride, 146mg of calcium chloride, 52mg of magnesium chloride, 342mg of dipotassium phosphate, and 1000mL purified water). The ratio of fiber to artificial saliva was 0.2g/1mL. An extract was prepared by being autoclaved at the temperature of 121±2 degrees centigrade for 1 hour, cooled down to 20-30 degrees centigrade, kept at room temperature and used within 24 hours.
Injection volume of the extract to the body weight was 50mL/kg, and the body weight measured just before injection. 50mL of the extract is equivalent to 10g fiber. The rats were starved for 18 hours or more since the day before the injection, and injected with the extract orally and compulsively for once by disposable syringes and oral sondes during 9:00-13:30. They were observed for 14 days after the injection.
They were observed continuously before the injection of the extract, at 5, 15, 30 minutes, 1, 2, and 4 hours after the injection, and once a day as from the following day. They were weighed before injection of the extract, and at 9:00-12:00 on 1, 3, 7, 10, $14^{th}$ day after injection. After all the observations, their organs and tissues in heads, chests and abdomens were researched whether normal or not.
As a result, no male and female rat in a group of the injection of the extract of the artificial saliva and a comparative group died during the experiments. The health conditions of male and female rats in both groups after the injection were confirmed as normal. The changes of the body weight on the injection group were going well and almost as same as that of the comparative group. The abnormal observations of the male and female rats in both groups were not shown in pathological anatomy.
In conclusion, it is suggested a lethal dose of the extract in this experiment is 10g fiber/kg or more that is equivalent as functional fiber.

6. Toxicity experiment by oral injection of solution dissolving the decomposer to rats (for once)

26 male rats and 26 female rats (Crj:CD(SD)IGS(SPF)) at the age of 5 weeks were housed for 1 week before experiment, and all rats were considered to be available and used to the experiment.
They were divided into each group according to their body weights measured before housed by a continuous stratified sampling method. Extra rats caused by the method were excluded from the experiment and euthanized. They were at the age of 6 weeks, 172-186g weigh (male), 130-151g weigh (female).
The test material of iron phthalocyanine tetracarboxylic acid was measured appropriate amount just before injection, and dissolved in purified water prepared to pH 10.2 by using of 1N sodium hydroxide. After dissolving, it was prepared to pH 7.11 by using of 1N hydrochloric acid, and injection solution was prepared by adding purified water so that the concentration of iron phthalocyanine tetracarboxylic acid was become to 100mg/mL by dilution. Following the

medical toxicity test guideline, the amount of injection was determined 2000mg/kg, technical upper limit of oral injection. A comparative group, which a comparative material of purified water in the Japanese Pharmacopoeia was injected, was also prepared. 10 male rats and 10 female rats were divided into two groups respectively.

Injection volume of the solution was determined by body weight in ratio of 20mL/kg, and the body weight measured just before the injection. The rats were starved for 18 hours or more since the day before the injection, and injected the solution orally and compulsively for once by disposable syringes (available from Terumo Corporation) and disposable oral sondes (available from Fuchigami Instruments Corporation). They were observed for 14 days after injection.

Their health conditions and life or death were observed continuously before the injection of the solution, at 5, 15, 30 minutes, 1, 2, and 4 hours after the injection, and once a day as from the following day. They were weighed before the injection of the solution, and at 9:00-12:00 on 1, 3, 7, 10, $14^{th}$ day after the injection. After all the observations, their organs and tissues in heads, chests and abdomens were researched whether normal or not.

As a result, no male and female rat in a group of the injection of the extract of the solution and a comparative group died during the experiments. The observation of the health conditions after injection revealed that all the male and female rats in the injection group had green feces in liquid state at 2 hours or more after the injection, and they had green feces at 1-$3^{rd}$ day after the injection. This was caused by color of the test material that is deep green powder. The health conditions of the male and female rats in the comparative group after the injection were confirmed as normal. The changes of the body weight on the injection group were going well and almost as same as that of the comparative group. The feces in liquid state was thought as transient diarrhea caused by the injection too much of the test material. The observation suggesting no toxicity of the solution was confirmed.

A minimum lethal dose of iron phthalocyanine tetracarboxylic acid in this experiment is 2000mg/kg or more, that is approximately calculated by a death rate during the observation.

7. Sensitization experiment in guinea pig skin

Male guinea pigs (Crj; Hartley) at the age of 5 weeks were divided into a comparative group of medium and a sensitization group of the functional fiber, and studied on the sensitization of the functional fiber to the skin by Maximization method. 151.88g of the functional fiber was shredded, and soaked in methanol of 10 times volume of fiber at room temperature. Methanol was reduced to prepare 5.482g of an extract.

In the comparative group of the medium, the guinea pigs were injected with the medium of 10% dimethylsulfoxide hypodermically to sensitize skin with dimethylsulfoxide, and then induced by 10, 1, 0.1% of the extract or the medium. As a result, no action of each induced part of the skin was observed.

In sensitization group of the extract, the guinea pigs were injected with 10% of the methanol extract of the functional fiber to sensitize, and induced by 10, 1, 0.1% of the extract or the medium.

As a result, on an induced part of the skin by 10% of the methanol extract of the functional fiber, some actions were observed. Mean of the score was 0.7 (at 24 hours after inducing) and 1.4 (at 48 hours after inducing), a positive rate was 10% (24 hours), and 40% (48 hours).

On an induced part of the skin by 1% of the methanol extract of the functional fiber, some red spots (score 1) were observed, but no positive example was observed.

On an induced part of the skin by 0.1% of the methanol extract of the functional fiber and an induced part of the skin by the medium, no action was observed. A positive rate at 48 hours after inducing revealed that the inducing by 10% of the methanol extract of the functional fiber had sensitization on the skin of middle-class (Grade III).

As the positive comparative group, guinea pigs were injected with 0.1% 2,4-dinitrochlorobenzene (DNCB) to sensitize, and induced by 0.1% DNCB or acetone. As a result, actions on all the guinea pigs were observed and a positive rate was 100% (at 48 hours after inducing), which revealed that the inducing by DNCB had sensitization on skin of heavy-class (Grade V).

Guinea pigs were injected with each material including adjuvant hypodermically, and at 7 days after injection, each material was stuck on the guinea pigs for 48 hours. At 21 days after injection, each material was stuck on the guinea pigs for 24 hours to sensitize transdermally, and actions of skin were scored at 24 and 48 hours after removing the materials. The scoring criteria on actions of skin of erythema or scab were as follows; no red spot : 0, slight red spot : 1, red spot : 2, middle red spot : 3, heavy red spot : 4. The scoring criteria on edema were as follows; no edema : 0, slight edema : 1, middle edema : 2, heavy edema : 3.

In conclusion, the inducing by the methanol extract of the functional fiber has sensitization on the guinea pig skin, the concentration of inducing by the extract was at least 10%, and the level of the sensitization by 10% thereof was middle-class (Grade III).

8. Sensitization experiment with rays in guinea pig skin

Male guinea pigs (Crj; Hartley) at the age of 5 weeks were studied on the sensitization with rays of the methanol extract of the functional fiber which was used on 7. sensitization experiment in guinea pig skin to the skin by Adjuvant and Strip method. The clipped backs of the guinea pigs were open spread by 0.1mL of each material which was used on 7. experiment, and after 30 minutes exposed to ultraviolet rays (about 10.2 Joules/$cm^2$). The procedures were repeated for 5 days and the guinea pigs were sensitized with rays. At 17th day since the last sensitization with rays, the clipped back skins were open spread symmetrically by 0.1mL of each material, and the guinea pigs were induced by exposing to ultraviolet rays (about 10.2Joules/$cm^2$) with their right halves of the bodies covered with aluminum foil. The reactions on the skins were evaluated at 24 and 48 hours after inducing by rays.

In sensitization with rays of the extract group, the guinea pigs were injected with 10% of the methanol extract of the functional fiber dissolving in dimethylsulfoxide to sensitize, and induced by 1% of the extract. As a result, some erythema (score 1) was observed, but no positive example was observed. All the guinea pigs of this group were evaluated as negative. In the comparative group of the medium, the guinea pigs were injected with to sensitize and induced by dimethylsulfoxide. As a result, no action of each induced part of the skin was observed. As the positive comparative group, the guinea pigs were injected with 5% 6-methylcoumarin (6-MC) to sensitize, and induced by 1% 6-MC. As a result, some erythema (score 2-4) were observed on the spots which were induced by 6-MC and exposed to ultraviolet rays, and all the guinea pigs of this group were evaluated as positive.

In conclusion, inducing by the methanol extract of the functional fiber doesn't have sensitization with rays on guinea pig skin.

9. Toxicity experiment with rays in guinea pig

Male guinea pigs (Crj; Hartley) at the age of 5 weeks were studied on the toxicity with rays of the methanol extract of the functional fiber which was used on 7. sensitization experiment in guinea pig skin by Morikawa et al. method. In sensitization with rays of the extract group, the clipped backs of the guinea pigs were open spread by 0.03mL of 10% of the methanol extract of the functional fiber dissolving in dimethylsulfoxide. In the comparative group of the medium, the clipped backs of the guinea pigs were open spread by 0.03mL of dimethylsulfoxide. As the positive comparative group, the clipped backs of the guinea pigs were open spread by 0.03mL of 0.05% 8-methoxypsoralen. After 30 minutes, they were exposed to ultraviolet rays (about 11.2Joules/$cm^2$). The reactions on the skins were evaluated at 24 and 48 hours after exposing to rays.

As a result, in sensitization with rays of the extract group and the comparative group of the medium, no action of each spread and non-spread part of the skin was observed, and all the guinea pigs of these groups were evaluated as negative. Whereas in the positive comparative group, some actions of score 4 were observed on all the spread part of the skins.

In conclusion, inducing by the methanol extract of the functional fiber doesn't have toxicity with rays on guinea pig.

10. Experiment on the ability to induce mutation by using bacterium

Whether the methanol extract of the functional fiber has an ability to induce mutation or not was examined by using Salmonella typhimurium TA100, TA1535, TA98, TA1537 as mouse typhoid bacillus and Escherichia coli WP2 uvrA as colon bacillus. The methanol extract was prepared as the residual by adding methanol of 10 times volume of the shredded functional fiber, stirring and extracting at room temperature for 24 hours, and reducing methanol with a rotary evaporator.

As a result, a number of return mutation colonies by the present treating of the extract increased twice less than that of the negative comparative group and didn't show the dose-dependent reaction on both the substituted base pair type and the shifted frame type regardless of the metabolic activation. Therefore the methanol extract of the functional fiber is considered not to have an ability to induce mutation under a condition of the present experiment.

11. Experiment on chromosome aberration by using mammalian cells

Whether the methanol extract of the functional fiber has an ability to induce chromosome aberration or not was examined. DMSO was used as medium on the experiment. The maximum test dose of the test material on the inhibition experiment of increasing cells was 5.0mg/mL. As a result of the inhibition experiment of increasing cells, a concentration in 50% inhibition experiment of increasing cells was 5.0mg/mL or more without a metabolic activation system treating in a short time (described as follows; -S9mix method). And the concentration thereof was 0.840mg/mL with a metabolic activation system treating in a short time (described as follows; +S9mix method). The precipitation of the test material was confirmed at the concentration of 0.313mg/mL or more. Therefore the maximum test dose in -S9mix method was

considered as 0.313mg/mL, the test doses of the experiment on chromosome aberration were prepared 3 doses diluted in a common ratio of 2. And the maximum test dose in +S9mix method was considered as 1.25mg/mL, the test doses thereof were prepared 4 doses diluted in a common ratio of 2.

As a result of treating in a short time, the appearance frequency of the cells and the polyploid cells whose chromosomes were abnormal structurally in -S9mix method was 5% or less in each test dose. However, the appearance frequency of the cells whose chromosomes were abnormal structurally in +S9mix method was increased dose-dependently in the concentration of 0.313mg/mL or more, and they were considered as positive. Furthermore, a continuous treating was not examined because they were considered as positive as a result of treating in a short time.

In the positive comparative group of each treating, the experiments were considered as being examined appropriately because the appearance frequency of the cells whose chromosomes were abnormal structurally was shown as appropriate values. In conclusion, the present test material was considered to be able to induce chromosome aberration on fibroblasts derived from Chinese hamster lung (CHL/IU cell).

The inhibition experiments of increasing cells were examined as follows. Treating in a short time No.1; in -S9mix method the cells were seeded in plastic laboratory dishes having a diameter of 60 mm (2 X $10^4$ cells (equivalent to 5.0mL of the culture solution)/dish), and cultured for 3 days. The maximum test dose in this method was considered as 5.0mg/mL, and the test doses were prepared the total 10 doses diluted the maximum test dose in a common ratio of 2 (0.010, 0.020, 0.039, 0.078, 0.156, 0.313, 0.625, 1.25, 2.5 and 5.0mg/mL). As the negative comparative, the comparative group of the medium (DMSO) was prepared. 50μL of the test solutions of the medium or each test dose were added to the laboratory dish per dose, and treated at the temperature of 37 degrees centigrade for 6 hours. After treating, removing the solutions in the laboratory dishes and washing cells in saline, 5mL of the new culture solutions were added. Furthermore the culture was continued for 18 hours. After the culture, removing the solutions in the laboratory dishes and washing cell surfaces in saline, the cells were fixed in 10% formalin solution. After fixing, the cells were stained by 0.1% crystal violet solution. The rate of the cell increasing was measured with the density meter of mono-layer cultured cells (monocellater: available from OLYMPUS OPTICAL CO., LTD) by observing light and shade of the stained cells. Under this measurement, the value of the cells in the comparative group of the medium was considered as 100%. The concentration of test material in 50% inhibition experiment of increasing cells was calculated approximately by this method.

Treating in a short time No.2; in +S9mix method, the same as -S9mix method, the cells were seeded and cultured for 3 days. The number of the laboratory dishes per dose and the test doses were prepared in the same condition as -S9mix method. 0.83mL of the culture solution was pulled out from the dish, added 0.83mL of S9mix, and S9mix diluted solution (the last concentration of S9 was 5%) was prepared. 50μL of the test solutions of the medium or each test dose were added to the laboratory dish per dose, and treated at the temperature of 37 degrees centigrade for 6 hours. The procedure after treating was the same as in -S9mix method.

The experiment on chromosome aberration was examined as follows. The experiment doses of the test material were determined by the inhibition experiment of increasing cells. As a result of the inhibition experiment of increasing cells, the concentration of test material in 50% inhibition experiment of increasing cells was 5.0mg/mL or more in - S9mix method treating in a short time. And the concentration thereof was 0.840mg/mL in +S9mix method treating in a short time.

The precipitation of the test material was confirmed at the concentration of 0.313mg/mL or more.

In conclusion, the test doses of the experiment on chromosome aberration treating in a short time were examined as follows. -S9mix method: 0.078, 0.156 and 0.313mg/mL (diluted in a common ratio of 2 or 3). +S9mix method: 0.156, 0.313, 0.625 and 1.25mg/mL (diluted in a common ratio of 2 or 4). As a result of the inhibition experiment of increasing cells, the maximum experiment dose of the test material was considered as 0.313mg/mL, and the test doses of the experiment were prepared 3 doses diluted the maximum test dose in a common ratio of 2. As the negative comparative, the non-treating group and the comparative group of the medium were prepared, and as the positive comparative, the mitomycin C (0.05μg/mL)-treating group was prepared.

Under this experiment, colcemid (available from GIBCO/Lot No. 1125546) was added to laboratory dishes for chromosome specimens to the last concentration of 0.2μg/mL at 2 hours before making specimens so that cell divisions were stopped in the middle of the division period. After 2 hours, the culture solutions in the laboratory dishes were moved into a conical bottom tube. Immediately 2mL of 0.25% trypsin solution was added to the laboratory dishes to exfoliate the cells, collected into a conical bottom tube described above, and centrifuged (1000rpm, 5 minutes). The resulting supernatants were removed, 5mL of 0.075M potassium chloride solutions were added, low osmotic pressure treating were performed in a thermostat of 37 degrees centigrade for 15 minutes, 0.5mL of cooling fixing solutions (mixture of cooling methanol and acetic acid in a ratio of 3 to 1) were added to fix partly, centrifuged (1000rpm, 5 minutes) immediately, and 5mL of new fixing solutions were added. The procedures were repeated 3 times to fix the cells perfectly. The fixed cells were prepared to the cell floating solution to be a little cloudy. And it was dropped onto the slide glass, dried by air, and stained by 1.7% Gimusa solution for about 15 minutes. Furthermore, the laboratory dishes prepared for the cell increasing rate were fixed and stained in the same method as the inhibition experiment of increasing cells described above, and the cell increasing rate was measured.

Under a microscope, 200 per each group (100 per a laboratory dish) of the well-spread figures in the middle

of the division period were observed, the types of the structural abnormality etc. and a number of the abnormal cells were recorded. At the same time, a number of the polyploid cell was recorded (they were recorded considering 37 or more chromosomes including 3 media as polyploid). Furthermore, to be observed objectively, the experiments were observed by blind tests.

The types of chromosome aberrations were classified as follows. However, gap was defined as a condition that non-stained part was on a vertical line of stained part, the width thereof was equal to or shorter than the width of a chromosome segment, and the form in non-stained part was clear. Break was defined as a condition that in case non-stained part was on a vertical line of the chromosome segment the width thereof was equal to or longer than the width of a segment and the form in non-stained part was clear, or the fragments were strayed from a vertical line of a chromosome or a chromosome segment. Exchange was defined as a mutual exchange by being broken at 2 or more part of a chromosome or a chromosome segment. The other structural abnormalities were defined as the other.

Structural abnormality
a chromosome segment break (ctb)
a chromosome segment exchange (radial exchange etc., cte)
a chromosome break (csb)
a chromosome exchange (dicentric, annular etc., cse)
others (fragmentation, frg)

The other chromosome abnormalities
Gap (g)

Abnormality in number
Polyploid, endoreduplication

As a result of treating in a short time, the appearance frequency of the cells and the polyploid cells whose chromosomes were abnormal structurally in -S9mix method was 5% or less in each test dose. However, the appearance frequency of the polyploid cells of chromosomes in +S9mix method was 5% or less in each test dose, the appearance frequency of the cells whose chromosomes were abnormal structurally in +S9mix method was increased dose-dependently (15.5% in 0.313mg/mL, 49.5% in 0.625mg/mL, and 73.0% in 1.25mg/mL). The precipitation of the test material was confirmed at the concentration of 0.313mg/mL or.more. The appearance frequency of the cells whose chromosomes were abnormal structurally was 16% in the positive comparative group of -S9mix method treating in a short time (MMC). The experiments were considered as being examined appropriately because the appearance frequency of the cells whose chromosomes were abnormal structurally was 37.5% in the positive comparative group of +S9mix method treating in a short time (DMN). Furthermore, the continuous treating was not examined because they were considered as positive as a result of treating in a short time. The test dose that the test material induces 20% of structural abnormality ($D_{20}$ value) was 0.35mg/mL in +S9mix method.

12. Toxicity experiment in cells

8g of the functional fiber was steam-sterilized under high pressure (121 degrees centigrade for 20 minutes). After cooling and drying, the functional fiber was added 80mL of the medium, and put a cap lightly. After being confirmed that the test material was soaked in the medium completely, it was put gently in the carbon dioxide incubator and incubated for 24 hours. Only the medium was pulled out from the glass bottle, and this medium was used as 100% extract of the test material. The extract was diluted with the medium to 100, 80, 70, 50, 30 and 10%. Furthermore, the concentrations of the extract of the test material described above were prepared 6 concentrations within an appropriate range so that $IC_{50}$ could be calculated from the results in pilot studies.

The functional fiber standard material or the blank fiber as the negative material (about 2X15mm) were put into the glass bottles and steam-sterilized under high pressure. After cooling and drying, the standard material or the negative material were added the medium and put caps lightly (10mL of the medium/1g of the standard material or the negative material). After the materials were put gently in the carbon dioxide incubator and incubated for 24 hours, only the media were pulled out from the glass bottles, and these media were used as 100% extract of the standard material or 100% extract of the negative material. The standard material A was diluted with the medium to 8.0, 3.0, 1.0, 0.5 and 0.1%. The standard material B was diluted with the medium to 90, 80, 70, 50 and 30%. 100% extract of the negative material was used as it was.

M05 medium excluding fetus bovine serum (FBS, available from Invitrogen Corporation, Lot No.A0282282) were prepared by adding 0.11g/L of sodium pyruvate (available from Wako Pure Chemical Industries, Ltd., Lot No.LDE0019), 2.2g/L of sodium bicarbonate (available from Kanto Chemical Co., Inc., Lot No.106G1268), 0.1mmol/L of MEM non-essential amino acids (available from Invitrogen Corporation, Lot No.1133557), 50U/mL of penicillin (available from Banyu Pharmaceutical Co., Ltd., Lot No.7QB03P) and 50μg/mL of streptomycin (available from Meiji Seika Kaisha, Ltd., Lot No.SSD468) to Eagle MEM medium (including Eagle equilibrium salt, including 0.292g/L of L-glutamine, available from Invitrogen Corporation, Lot No.1101728). M05 medium was prepared by adding 5v/v% FBS to M05

medium excluding FBS.

Cell culture was performed by putting gently on the condition that the temperature was 37.0±1.0 degrees centigrade (actual temperature : 36.9-37.4 degrees centigrade), the concentration of $CO_2$ was 5.0±0.5% (actual concentration : 4.8-5.1%), under humidity in the carbon dioxide incubator. The inherited operation was performed on the condition that the cell density was about 30-70% of the area of a flask base. The medium was removed from the flask, and the flask was rinsed lightly with Dulbecco orthophosphate buffer excluding $Ca^{2+}$ and $Mg^{2+}$. After removing PBS(-) by suction, the amount of PBS(-) including 0.05% trypsin and 0.02% EDTA·2Na (Dojindo laboratories, Lot No.KC159)(trypsin/EDTA solution) as small as cells were soaked, was added thereto, and put gently in the carbon dioxide incubator. After confirming that the cells were almost come off from the flask base under observation through a microscope, the cells were collected by adding the medium, moved into the centrifuge tube and centrifuged at about 80 rpm for 2 minutes. The cell-dispersing suspension was prepared by removing the supernatants, adding new medium and taking the cells apart. The cells were diluted with the medium to 1/3 - 1/10 of the cell density before the inherited operation, and seeded in a new flask.

The cells were come off by the method of the inherited operation, the cell-dispersing suspension prepared by diluting with the medium to 1000cells/mL was added 0.1mL/well into 6-well plates which were added 3mL of the medium previously, and cultured in the carbon dioxide incubator for 24 hours. After removing the medium, 3mL of each concentration of the extract of the test material, the standard material A, the standard material B and the negative material (only 100% extract) was added. And 4 wells added only the medium were prepared as the control group. 4 wells per a concentration were used (n=4). After adding each extract and culturing in the carbon dioxide incubator for 7 days, the medium in each well was removed and the wells were washed with 3mL of PBS(-)/well. After adding 3mL of methanol into each well and putting gently for 10 minutes to fix the cells, methanol was removed, 3mL of 5% Gimusa solution diluted with a phosphate buffer (pH6.4) was added, and the wells were put gently for 10 minutes to stain the colonies. After washing each well with the purified water once and drying, a number of the colonies which were considered to consist of 50 or more cells were measured macroscopically or through an actual microscope.

After V79 cells as one of the cell lines derived from male Chinese hamster lung were cultured (100cells/well) in 6-well plates for 24 hours, the extracts of the test material (the functional fiber) and each comparative material (the standard material A: a polyurethane film including 0.1% zinc diethyldithiocarbamate (ZDEC), the standard material B: a polyurethane film including 0.25% zinc dibuthyldithiocarbamate (ZDBC), and the negative material: a high-density polyethylene film) was added 4 wells respectively, and cultured for 7 days. The formed colonies were measured, and the concentration of the extract in case these colonies were half of the colonies cultured in the medium only in the same method (the control group) ($IC_{50}$) was calculated. The ability of forming colonies in the control group was 95%, which was considered as well. A statistical significant difference in the colonies between the control group and the wells added 100% extract of the negative material was not confirmed. $IC_{50}$ of the standard material A was 1.32%, and that of the standard material B was 68.92%, which cleared the valid criteria of the present test respectively.

Compared with the size of the colonies in the well added the extract of the functional fiber and in control group, the colonies by using the extract of high concentration (70% or more) showed a tendency to be small, which suggested a little influence on the ability of the increasing cells. However, there was no difference in the number of the formed colonies between the extract of the functional fiber and the control group, $IC_{50}$ of the extract of the functional fiber was 100% or more, which confirmed no influence on the ability of the forming colonies.

In conclusion, it was considered that there was no inhibitive action for the forming colonies by using the extract of the functional fiber on a condition of the present experiment.

Next, the desirable experimental examples of the antiallergenic feathers, the composition and the feather product containing the feathers applicable to the present invention were explained.

The present invention is the antiallergenic feathers carrying the compounds of metal phthalocyanine shown as the formula described previously (II) as one of the derivatives of metal phthalocyanine described previously (described the compounds of metal phthalocyanine (II) as follows). The amount of the compounds of metal phthalocyanine (II) which the feathers described previously has is desirably 0.1 mass% or more and 10 mass% or less, more desirably 0.3 mass% or more and 5 mass% or less, much more desirably 0.5 mass% or more and 3 mass% or less as contrasted with the feathers weight described previously. It is because in case the amount of the compounds of metal phthalocyanine (II) or phthalocyanate thereof is 0.1 mass% or more and 10 mass% or less, the antiallergenic feathers have an excellent effect on the adsorption to allergens derived from tick.

As the material of the antiallergenic feathers described previously, feathers picked from duck, goose, wild duck, mallard, Rouen duck, Chinese duck, Beijing duck, goose such as Pilgrim goose or Emden goose, Greylag goose is considered as applicable. And, as the material of the antiallergenic feathers described previously, feathers such as large feathers, feathers with a stalk on the part of the wing; down such as hair on the chest, fluff; small feathers can be used on the present invention.

As the phthalocyanate of the metal phthalocyanine (II), corresponding salt with inorganic base and corresponding salt with organic base are mentioned for example. Preferable examples of the corresponding salt with inorganic base

are an alkali metal salt such as sodium salt, potassium salt; an alkaline earth metal salt such as calcium salt, magnesium salt; copper(II) salt; ammonium salt. Preferable examples of the corresponding salt with organic base are corresponding salts with trimethylamine, triethylamine, pyridine, picoline, ethanolamine, diethanolamine, triethanolamine, dicyclohexylamine.

It is preferable that M of the metal phthalocyanine (II) or the phthalocyanate thereof in the above formula (II) is Co or Fe. It is much preferable that $R^1$, $R^2$, $R^3$, $R^4$ of the metal phthalocyanine (II) or the phthalocyanate thereof in the above formula (II) are same and are $SO_3H$ groups, and n1, n2, n3, n4 of the metal phthalocyanine (II) or the phthalocyanate thereof in the above formula (II) are same and are 1. Concrete examples of such metal phthalocyanine (II) or the phthalocyanate thereof are mentioned as following structural formulae.

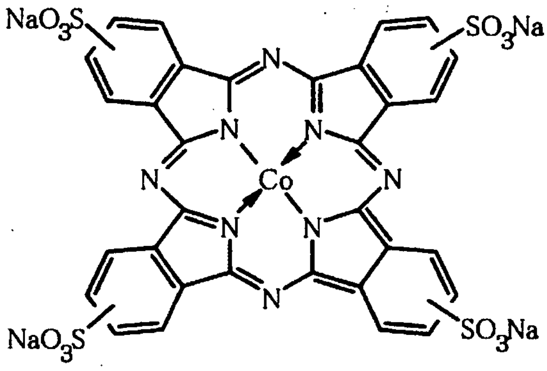

・・・(IX)

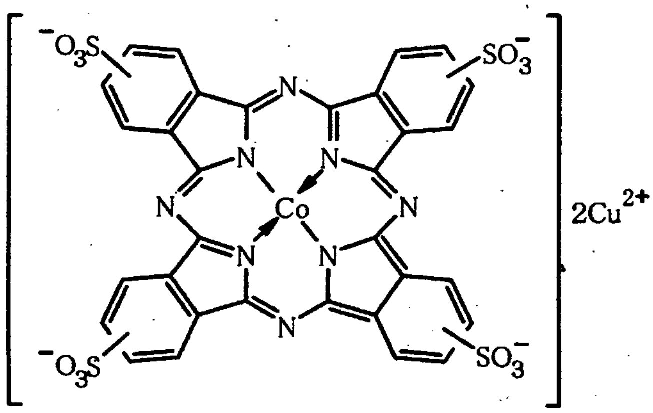

・・・(X)

The phthalocyanate of the metal phthalocyanine (II) is much preferably sodium salt or copper(II) salt.

The antiallergenic feathers carrying the metal phthalocyanine (II) or the phthalocyanate thereof of the present invention are prepared as following method for example. First, the feathers are pretreated. As the pretreatment, a dust removal treatment which removes the clay and sands or another impurities from the feathers as material; a dust removal treatment before cleaning which removes dusts or dirt that is unremovable pebbles, sands and fatty acid from the feathers through the cleaning; a cleaning treatment by water at normal temperature or warm water; are mentioned.

Next, the pretreated feathers are soaked in aqueous solution of the metal phthalocyanine (II) or the phthalocyanate thereof at 80 degrees centigrade or more and 100 degrees centigrade or less for 30 minutes to 2 hours. The feathers are washed with water and dried, and then the antiallergenic feathers carrying the metal phthalocyanine (II) or the phthalocyanate thereof can be prepared.

The amount of the metal phthalocyanine (II) or the phthalocyanate thereof in the aqueous solution is preferably 0.01 mass% or more and 10 mass% or less to weight of the feathers, much preferably 0.1 mass% or more and 5 mass% or less.

An acid generally used in an acid stain method such as formic acid, acetic acid, hydrochloric acid, phosphoric acid, citric acid may be included in the aqueous solution of the metal phthalocyanine (II) or the phthalocyanate thereof. The acid is used in order to cationize an amino group of the amino acid constituting the feathers and to make the solubility of the metal phthalocyanine (II) or the phthalocyanate thereof poor, so that the metal phthalocyanine (II) or the phthalocyanate thereof can be easily carried to the feathers.

The antiallergenic feathers carrying the metal phthalocyanine (II) or the phthalocyanate thereof may be treated by a mordant or a dye-fixing agent. As the mordant, a tannic acid; an aluminum salt such as aluminum acetate, ammonium alum, burnt alum; a chromic acid such as chromium acetate, chromium alum; a ferrate such as ferric sulfamate, iron pyroligneous acid, ferrous chloride, ferrous sulfate; a tin salt such as sodium stannate, stannous chloride; a copper salt such as copper acetate; a barium salt such as barium chloride; can be used. As the dye-fixing agent, a copper salt such as copper(II) sulfate; an aluminum salt; a quaternary ammonium salt; can be used. When the antiallergenic feathers are treated by the mordant, free $SO_3H$ group or free COOH group in the metal phthalocyanine (II) can form sulfate or carboxylate, so the metal phthalocyanine (II) or the phthalocyanate thereof is carried strongly to the feathers. Therefore, when the antiallergenic feathers carrying the metal phthalocyanine (II) or the phthalocyanate thereof is washed, the metal phthalocyanine (II) or the phthalocyanate thereof can be maintained to carry onto the feathers for a long period of time.

The other present invention is a composition comprising the antiallergenic feathers carrying metal phthalocyanine(II) or the phthalocyanate thereof. The composition may be processed into moldings combining other materials.

The other present invention is feather product comprising the antiallergenic feathers carrying metal phthalocyanine(II) or the phthalocyanate thereof. As the feather products, bedclothes such as feather bedding; clothes such as feather jacket; are mentioned.

The antiallergenic feathers of the present invention will be more clearly understood with reference to the following examples 2 and 3. Further, a bulk on these experiments was measured in the experiment room at the temperature of 20 degrees centigrade and at the relative humidity of 65% following JIS L 1903 method of the feather experiment.

(Example 2)

Goose feathers with treating of removing dusts previously, treating of removing dusts before cleaning and treating of cleaning in water (85 mass% of down, 15 mass% of small feather) (153mm of the bulk) were prepared.

Whereas, 0.5 mass% (as contrasted with the feather weight) of cobalt phthalocyanine polysulfonic acid sodium salt as described formula (IX) previously and 2 mass% (as contrasted with the feather weight) of formic acid were dissolved in water of 30 times volume of them, and the reagent solution was prepared.

The feathers described previously were soaked in this reagent solution, and treated at the temperature of 97 degrees centigrade for 50 minutes. Most of cobalt phthalocyanine polysulfonic acid sodium salt was adsorbed to the feathers.

After that, the feathers were washed in water to remove the left reagent solution. Following, dehydrated and dried, and the antiallergenic feathers carrying cobalt phthalocyanine polysulfonic acid sodium salt (153mm of the bulk) was prepared. Comparing the feathers obtained in this way with the feathers before carrying the compounds of metal phthalocyanine (II) or phthalocyanate thereof, there was no change in the touch and the bulk.

(Example 3)

The goose feathers with treating of removing dusts previously, treating of removing dusts before cleaning and treating of cleaning in water (85 mass% of down, 15 mass% of small feather) were prepared.

Whereas, 0.5 mass% (as contrasted with the feather weight) of cobalt phthalocyanine polysulfonic acid sodium salt as described formula (IX) previously and 2 mass% (as contrasted with the feather weight) of formic acid were dissolved in water of 30 times volume of them, and the reagent solution was prepared. The feathers described previously were soaked in this reagent solution, and treated at the temperature of 97 degrees centigrade for 50 minutes. After that, the feathers were washed in water to remove the left reagent solution. Following, it was dehydrated and dried.

And 1 mass% (as contrasted with the feather weight) of copper sulfate 5 hydrate was dissolved in water of 30 times volume of them, and the reagent solution was prepared. The feathers described previously were soaked in this reagent solution, and treated at the temperature of 30 degrees centigrade for 30 minutes. As a result, copper ion bonded to cobalt phthalocyanine polysulfonic acid, and insoluble copper salt shown as a formula (X) described previously was formed.

After that, the feathers were washed in water to remove the left reagent solution. Following, dehydrated and dried, and the antiallergenic feathers carrying the compounds of metal phthalocyanine copper salt (X) were prepared. Comparing the feathers obtained in this way with the feathers before carrying the compounds of metal phthalocyanine (II) or phthalocyanate thereof, there was no change in the touch and the bulk.

(Evaluation)

1. Experiment on the adsorption ability to the tick allergen

The antiallergenic feathers carrying the compounds of metal phthalocyanine (II) or phthalocyanate thereof obtained on example 2 and 3 were prepared as a test sample, and the adsorption ability of the tick allergen was measured as follows. As the comparative sample, the feathers without treating in the reagent solution on example 2 and 3, in short, the goose feather with treating of removing dusts previously, treating of removing dusts before cleaning and treating of cleaning in water (85 mass% of down, 15 mass% of small feather) were used.

2mg of each sample was put into 200μL of tick allergen antigen solution (available from ASAHI BREWERIES, LTD., purified tick allergen rDer2 (goods name); 1μg/mL), and soaked at the temperature of 25 degrees centigrade for 1 hour. The solutions after removing the samples were centrifuged (10000rpm, 3 minutes), and the supernatants (100μL) were measured on ELISA method.

Measurement method by enzyme-linked immuno-sorbent assay (ELISA method)

(1) Coating of the antigen

The supernatant described previously was put into a microplate (96-well plate made of vinyl chloride, available from Dynatech Co., Ltd.) at the ratio of 100μL per well, and treated at the temperature of 25 degrees centigrade for 2 hours. After that, the supernatant described previously was removed from each well using a micropipette. The wells were washed in PBS solution three times.

(2) Blocking with BSA

1 w/v% of BSA solution (available from VECTOR LABORATORIES, BOVINESERUMALBUMIN (goods name)) was put into the microplate described previously at the ratio of 100μL per well, and treated at the temperature of 25 degrees centigrade for 1 hour. After that, the supernatant described previously was removed from each well using the micropipette. The wells were washed in 0.05% Tween-PBS solution once.

(3) Reaction of primary antibodies to the .antigen

Tick allergen antibody solution (available from ASAHI BREWERIES, LTD., anti-Derf2 monoclonal antibody (goods name); 5μg/mL) was put into the microplate described previously at the ratio of 100μL per well, and treated at the temperature of 25 degrees centigrade for 2 hours.

(4) Reaction of the enzyme-labeled second antibody

Biotin-labeled anti-mouse IgG (H+L) antibody solution (available from VECTOR LABORATORIES, BIOTINYLATED ANTI-MOUSE IgG (H+L) (goods name); 1μg/mL) was put into the microplate described previously at the ratio of 100μL per well, and treated at the temperature of 25 degrees centigrade for 2 hours. After that, the antibody solution described previously was removed from each well using the micropipette. The wells were washed in 0.05% Tween-PBS solution once.

(5) Modification by AVIDIN

Avidin solution (available from VECTOR LABORATORIES, ALKALINE PHOSPHATASE AVIDIN D (goods name); 100 unit) was put into the microplate described previously at the ratio of 100μL per well, and treated at the temperature of 25 degrees centigrade for 0.5 hours. After that, the supernatant described previously was removed from each well using the micropipette. The wells were washed in 0.05% Tween-PBS solution three times.

(6) Reaction to a developing matrix

PNPP solution (available from VECTOR LABORATORIES, P-NITROPHENYL PHOSPHATE (goods name); 500μg/mL) was put into the microplate described previously at the ratio of 100μL per well, and treated at the temperature of 25 degrees centigrade for 10 minutes. After that, 5N NaOH aqueous solution was put at the ratio of 100μL per well, and the reaction was stopped.

(7) Measurement

The absorbance at 405 nm or more was measured by a microplate reader (available from BIO-RAD LABORATORIES, MICROPLATE READER Model 550 (goods name)), and the concentration of the tick allergen in each sample was quantified. The adsorption rate of the samples to the tick allergen was calculated according to the formula described below using the concentration of the tick allergen before soaking samples (1μg/mL) and the concentration of the tick allergen after soaking samples.

Adsorption rate (%) = [(Concentration of tick allergen after soaking samples: μg/mL)/(Concentration of tick allergen before soaking samples (1μg/mL)) X 100

Table 5

| | adsorption rate |
|---|---|
| Example 2 | 93% |
| Example 3 | 91% |
| Comparative | 22% |

As the obtained results were obvious, it was confirmed that the antiallergenic feathers with the derivatives of metal phthalocyanine of the present invention did not require a tick repellent and had excellent absorption ability to the tick allergen. Therefore, the feathers of the present invention do not require a tick repellent and has an excellent antiallergenic effect.

Industrial Applicability

The allergen decomposers of the present invention can be used as medicines because the efficacy thereof to decompose allergens by the effect of the derivatives of metal phthalocyanine as active principles was confirmed experimentally, and safety pharmacology thereof was also confirmed experimentally.

Furthermore, the antiallergenic feathers of the present invention can be applied to the composition and the feather product containing thereof because they also have an effect on adsorbing the allergens.

## Claims

1. A method for decomposing an allergen placing an allergen decomposer in an environment, wherein the allergen decomposer comprises a metal phthalocyanine derivative represented by the following formula (II)

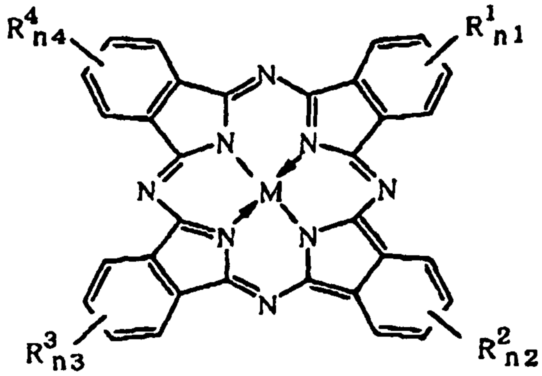

· · · (II)

in the formula (II), M is a metal selected from the group consisting of Fe, Co, Mn, Ti, V, Ni, Cu, Zn, Mo, W, Os; $R^1{}_{n1}$, $R^2{}_{n2}$, $R^3{}_{n3}$ and $R^4{}_{n4}$ are substituents that are same or different to each other and that are COOH group or $SO_3H$ group; n1, n2, n3, n4 are same or different to each other and are 0 to 4 of numbers of substituents that satisfy $1 \leq n1 + n2 + n3 + n4 \leq 8$.

2. The method for decomposing the allergen according to claim 1, wherein the metal phthalocyanine derivative is metal phthalocyanine dicarboxylic acid, metal phthalocyanine tetracarboxylic acid, metal phthalocyanine octacarboxylic acid, metal phthalocyanine disulfonic acid, metal phthalocyanine tetrasulfonic acid, metal phthalocyanine octasulfonic acid, or carboxylate or sulfonate thereof.

3. The method for decomposing the allergen according to claim 1, wherein the allergen is a protein allergen.

4. The method for decomposing the allergen according to claim 1, wherein the metal phthalocyanine derivative is carried or mixed to the carrier.

5. An antiallergenic feather carrying an allergen decomposer of active ingredient, wherein the allergen decomposer comprises a metal phthalocyanine derivative represented by the following formula (II)

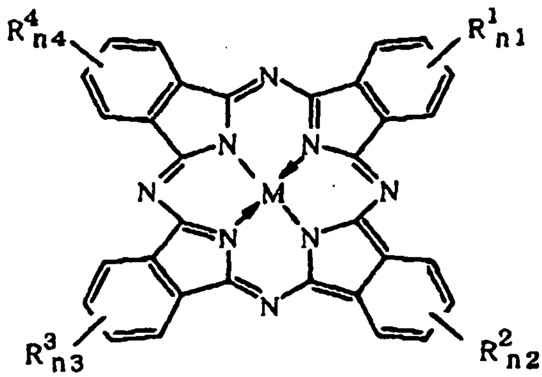

· · · (II)

(in the formula (II), M is a metal selected from the group consisting of Fe, Co, Mn, Ti, V, Ni, Cu, Zn, Mo, W, Os; $R^1{}_{n1}$, $R^2{}_{n2}$, $R^3{}_{n3}$ and $R^4{}_{n4}$ are substituents that are same or different to each other and that are COOH group or $SO_3H$ group; n1, n2, n3, n4 are same or different to each other and are 0 to 4 of numbers of substituents that satisfy $1 \leq n1 + n2 + n3 + n4 \leq 8$).

6. The antiallergenic feather according to claim 5, wherein the amount of the metal phthalocyanine derivative is 0.1 mass % or more and 10 mass % or less to weight of the feather.

7. The antiallergenic feather according to claim 5, wherein a raw of said feather is one selected from large feather of

a stalk on a wing, down, fluff and small feather of a duck or/and goose.

**8.** A feather antiallergenic product including said antiallergenic feather according to claim 5.

**9.** The antiallergenic feather product according to claim 8, wherein said product is one selected from a bedding and jacket.

**10.** Use of a fiber material carrying an allergen decomposer of active ingredient, wherein the allergen decomposer comprises a metal phthalocyanine derivative represented by the following formula (II)

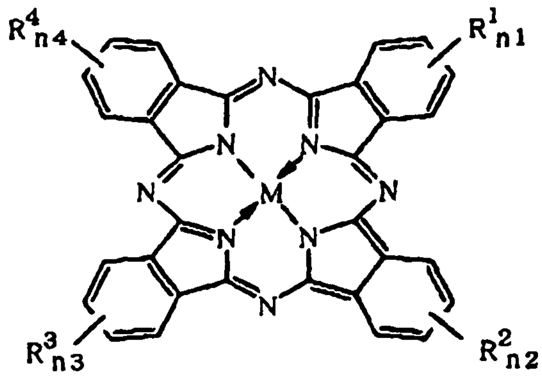

· · · (II)

(in the formula (II), M is a metal selected from the group consisting of Fe, Co, Mn, Ti, V, Ni, Cu, Zn, Mo, W, Os; $R^1_{n1}$, $R^2_{n2}$, $R^3_{n3}$ and $R^4_{n4}$ are substituents that are same or different to each other and that are COOH group or $SO_3H$ group; n1, n2, n3, n4 are same or different to each other and are 0 to 4 of numbers of substituents that satisfy $1 \leq n1 + n2 + n3 + n4 \leq 8$) as antiallergenic fiber material.

**11.** Use of the fiber material according to claim 10, wherein a raw of said fiber materials is one selected from cellulose fiber of cotton, hemp, or rayon; protein fiber of wool or silk; polyamide fiber, polyester fiber, polyacryl fiber, polyvinyl alcohol fiber, polyvinyl chloride fiber, polyvinylidene chloride fiber, polyolefin fiber and polyurethane fiber.

**12.** Use of the fiber material according to claim 10 for obtaining an antiallergenic fiber product.

**13.** Use of the fiber material according to claim 12, wherein said antiallergenic fiber product is one selected from a cloth, bedding, curtain, wallpaper, carpet, air filter and knit.

## Patentansprüche

**1.** Verfahren zum Zersetzen eines Allergens durch Platzieren eines Allergenzersetzers in einer Umgebung, wobei der Allergenzersetzer ein Metallphthalocyanin-Derivat umfasst, das durch die folgende Formel (II) dargestellt wird

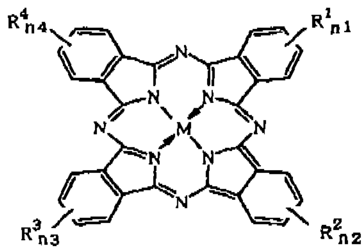

· · · (II)

wobei in Formel (II) M ein Metall ist, das aus der Gruppe ausgewählt ist, die aus Fe, Co, Mn, Ti, V, Ni, Cu, Zn, Mo,

W, Os besteht, $R^1_{n1}$, $R^2_{n2}$, $R^3_{n3}$ und $R^4_{n4}$ Substituenten sind, die gleich oder verschieden sind und eine COOH-Gruppe oder $SO_3H$-Gruppe sind; n1, n2, n3, n4 gleich oder verschieden sind und Zahlen von Substituenten von 0 bis 4 entsprechen, für die gilt: $1 \leq n1 + n2 + n3 + n4 \leq 8$.

**2.** Verfahren zum Zersetzen des Allergens gemäß Anspruch 1, wobei es sich bei dem Metallphthalocyanin-Derivat um Metallphthalocyanindicarbonsäure, Metallphthalocyanintetracarbonsäure, Metallphthalocyaninoctacarbonsäure, Metallphthalocyanindisulfonsäure, Metallphthalocyanintetrasulfonsäure, Metallphthalocyaninoctasulfonsäure oder ein Carboxylat oder Sulfonat davon handelt.

**3.** Verfahren zum Zersetzen des Allergens gemäß Anspruch 1, wobei das Allergen ein Proteinallergen ist.

**4.** Verfahren zum Zersetzen des Allergens gemäß Anspruch 1, wobei das Metallphthalocyanin-Derivat geträgert oder mit dem Träger gemischt ist.

**5.** Antiallergene Feder, die einen Allergenzersetzer als Wirkstoff trägt, wobei der Allergenzersetzer ein Metallphthalocyanin-Derivat umfasst, das durch die folgende Formel (II) dargestellt wird

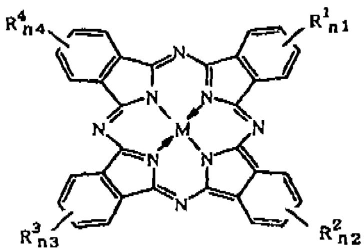

· · · (II)

(wobei in Formel (II) M ein Metall ist, das aus der Gruppe ausgewählt ist, die aus Fe, Co, Mn, Ti, V, Ni, Cu, Zn, Mo, W, Os besteht, $R^1_{n1}$, $R^2_{n2}$, $R^3_{n3}$ und $R^4_{n4}$ Substituenten sind, die gleich oder verschieden sind und eine COOH-Gruppe oder $SO_3H$-Gruppe sind; n1, n2, n3, n4 gleich oder verschieden sind und Zahlen von Substituenten von 0 bis 4 entsprechen, für die gilt: $1 \leq n1 + n2 + n3 + n4 \leq 8$).

**6.** Antiallergene Feder gemäß Anspruch 5, wobei die Menge des Metallphthalocyanin-Derivats 0,1 Massen-% oder mehr und 10 Massen-% oder weniger beträgt, bezogen auf das Gewicht der Feder.

**7.** Antiallergene Feder gemäß Anspruch 5, wobei das Rohmaterial für die Feder aus Kielfedern, Daunen, Flaumfedern und kleinen Federn einer Ente oder/und Gans ausgewählt ist.

**8.** Antiallergenes Federprodukt, das die antiallergene Feder gemäß Anspruch 5 enthält.

**9.** Antiallergenes Federprodukt gemäß Anspruch 8, wobei das Produkt aus Bettzeug und einer Jacke ausgewählt ist.

**10.** Verwendung eines Fasermaterials, das einen Allergenzersetzer als Wirkstoff trägt, wobei der Allergenzersetzer ein Metallphthalocyanin-Derivat umfasst, das durch die folgende Formel (II) dargestellt wird

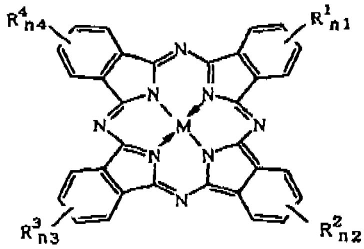

· · · (II)

(wobei in Formel (II) M ein Metall ist, das aus der Gruppe ausgewählt ist, die aus Fe, Co, Mn, Ti, V, Ni, Cu, Zn,

Mo, W, Os besteht, $R^1_{n1}$, $R^2_{n2}$, $R^3_{n3}$ und $R^4_{n4}$ Substituenten sind, die gleich oder verschieden sind und eine COOH-Gruppe oder $SO_3H$-Gruppe sind; n1, n2, n3, n4 gleich oder verschieden sind und Zahlen von Substituenten von 0 bis 4 entsprechen, für die gilt: $1 \leq n1 + n2 + n3 + n4 \leq 8$), als antiallergenes Fasermaterial.

**11.** Verwendung des Fasermaterials gemäß Anspruch 10, wobei das Rohmaterial für die Fasermaterialien aus Cellulosefasern, wie Baumwolle, Hanf oder Rayon, Proteinfasern, wie Wolle oder Seide, Polyamidfasern, Polyesterfasern, Polyacrylfasern, Polyvinylalkoholfasern, Polyvinylchloridfasern, Polyvinylidenchloridfasern, Polyolefinfasern und Polyurethanfasern ausgewählt ist.

**12.** Verwendung des Fasermaterials gemäß Anspruch 10 zur Gewinnung eines antiallergenen Faserprodukts.

**13.** Verwendung des Fasermaterials gemäß Anspruch 12, wobei das antiallergene Faserprodukt aus einem Tuch, Bettzeug, Vorhang, Tapete, Teppich, Luftfilter und Maschenware ausgewählt ist.

## Revendications

**1.** Procédé pour décomposer un allergène en plaçant un agent décomposant d'allergène dans un environnement, dans lequel l'agent décomposant d'allergène comprend un dérivé de phtalocyanine métallique représenté par la formule (II) suivante

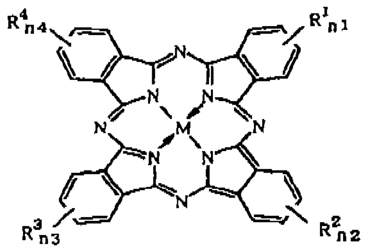

· · · (II)

où, dans la formule (II), M est un métal choisi dans le groupe consistant en Fe, Co, Mn, Ti, V, Ni, Cu, Zn, Mo, W, Os ; $R^1_{n1}$, $R^2_{n2}$, $R^3_{n3}$ et $R^4_{n4}$ sont des substituants identiques ou différents qui sont des groupes COOH ou $SO_3H$ ; n1, n2, n3, n4 sont identiques ou différents et sont des nombres de substituants valant de 0 à 4 et satisfaisant la relation $1 \leq n1 + n2 + n3 + n4 \leq 8$.

**2.** Procédé pour décomposer un allergène selon la revendication 1, dans lequel le dérivé de phtalocyanine métallique est un acide de phtalocyaninedicarboxylique métallique, un acide de phtalocyaninetétracarboxylique métallique, un acide de phtalocyanineoctacarboxylique métallique, un acide de phtalocyaninedisulfonique métallique, un acide de phtalocyaninetétrasulfonique métallique, un acide de phtalocyanineoctasulfonique métallique, ou un carboxylate ou sulfonate de ceux-ci.

**3.** Procédé pour décomposer un allergène selon la revendication 1, dans lequel ledit allergène est un allergène protéique.

**4.** Procédé pour décomposer un allergène selon la revendication 1, dans lequel le dérivé de phtalocyanine métallique est supporté ou mélangé avec un support.

**5.** Plume anti-allergène portant un agent décomposant d'allergène comme principe actif, dans laquelle l'agent décomposant d'allergène comprend un dérivé de phtalocyanine métallique représenté par la formule (II) suivante

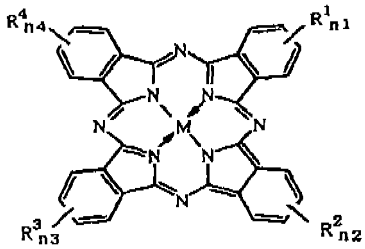

· · · (I I)

(où, dans la formule (II), M est un métal choisi dans le groupe consistant en Fe, Co, Mn, Ti, V, Ni, Cu, Zn, Mo, W, Os ; $R^1_{n1}$, $R^2_{n2}$, $R^3_{n3}$ et $R^4_{n4}$ sont des substituants identiques ou différents qui sont des groupes COOH ou $SO_3h$ ; n1, n2, n3, n4 sont identiques ou différents et sont des nombres de substituants valant de 0 à 4 et satisfaisant la relation $1 \leq n1 + n2 + n3 + n4 \leq 8$).

**6.** Plume anti-allergène selon la revendication 5, dans laquelle la quantité du dérivé de phtalocyanine métallique est au moins 0,1 pour cent massique et au plus 10 pour cent massique, par rapport au poids de la plume.

**7.** Plume anti-allergène selon la revendication 5, dans laquelle la matière première de ladite plume est choisie parmi les pennes, les plumes de duvet, les plumules et les petites plumes d'un canard ou/et d'une oie.

**8.** Produit de plume anti-allergène comprenant ladite plume anti-allergène selon la revendication 5.

**9.** Produit de plume anti-allergène selon la revendication 8, dans lequel ledit produit est choisi parmi un linge de lit et un manteau.

**10.** Utilisation d'un matériau fibreux portant un agent décomposant d'allergène comme principe actif, dans laquelle l'agent décomposant d'allergène comprend un dérivé de phtalocyanine métallique représenté par la formule (II) suivante

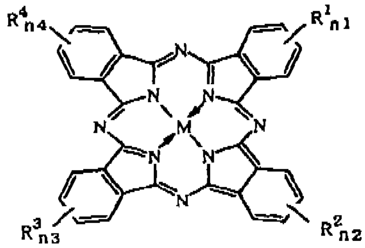

· · · (I I)

(où, dans la formule (II), M est un métal choisi dans le groupe consistant en Fe, Co, Mn, Ti, V, Ni, Cu, Zn, Mo, W, Os ; $R^1_{n1}$, $R^1_{n2}$, $R^3_{n3}$ et $R^4_{n4}$ sont des substituants identiques ou différents qui sont des groupes COOH ou $SO_3H$ ; n1, n2, n3, n4 sont identiques ou différents et sont des nombres de substituants valant de 0 à 4 et satisfaisant la relation $1 \leq n1 + n2 + n3 + n4 \leq 8$) comme matériau fibreux anti-allergène.

**11.** Utilisation du matériau fibreux selon la revendication 10, dans laquelle la matière première desdits matériaux fibreux est choisie parmi les fibres cellulosiques de coton, de chanvre ou de viscose, les fibres protéiques de laine ou de soie ; les fibres de polyamide, les fibres de polyester, les fibres polyacryliques, les fibres d'alcool polyvinylique, les fibres de polychlorure de vinyle, les fibres de polychlorure de vinylidène, les fibres de polyoléfine et les fibres de polyuréthane.

**12.** Utilisation du matériau fibreux selon la revendication 10 pour obtenir un produit fibreux anti-allergène.

**13.** Utilisation du matériau fibreux selon la revendication 12, dans laquelle le produit fibreux anti-allergène est choisi parmi un chiffon, un linge de lit, un rideau, du papier peint, un tapis, un filtre à air et une bonneterie.

Fig. 1

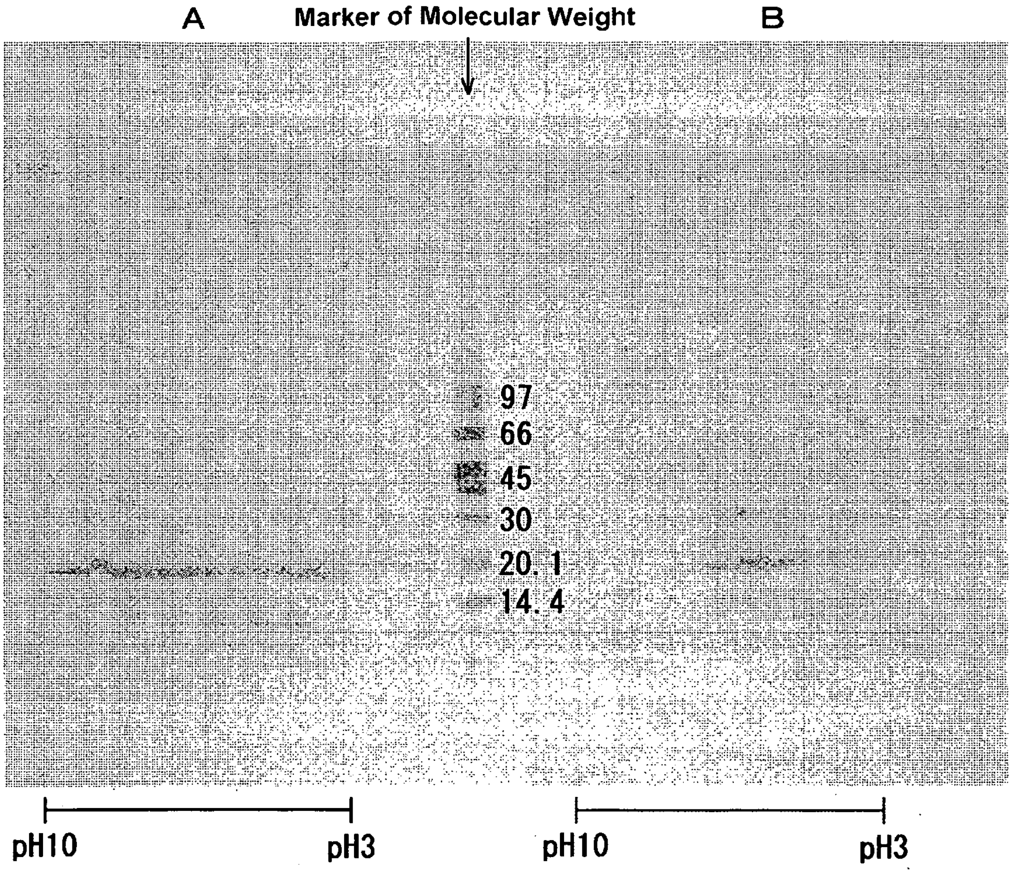
A
Marker of Molecular Weight
B
97
66
45
30
20. 1
14. 4
pH10
pH3
pH10
pH3

## Fig. 2

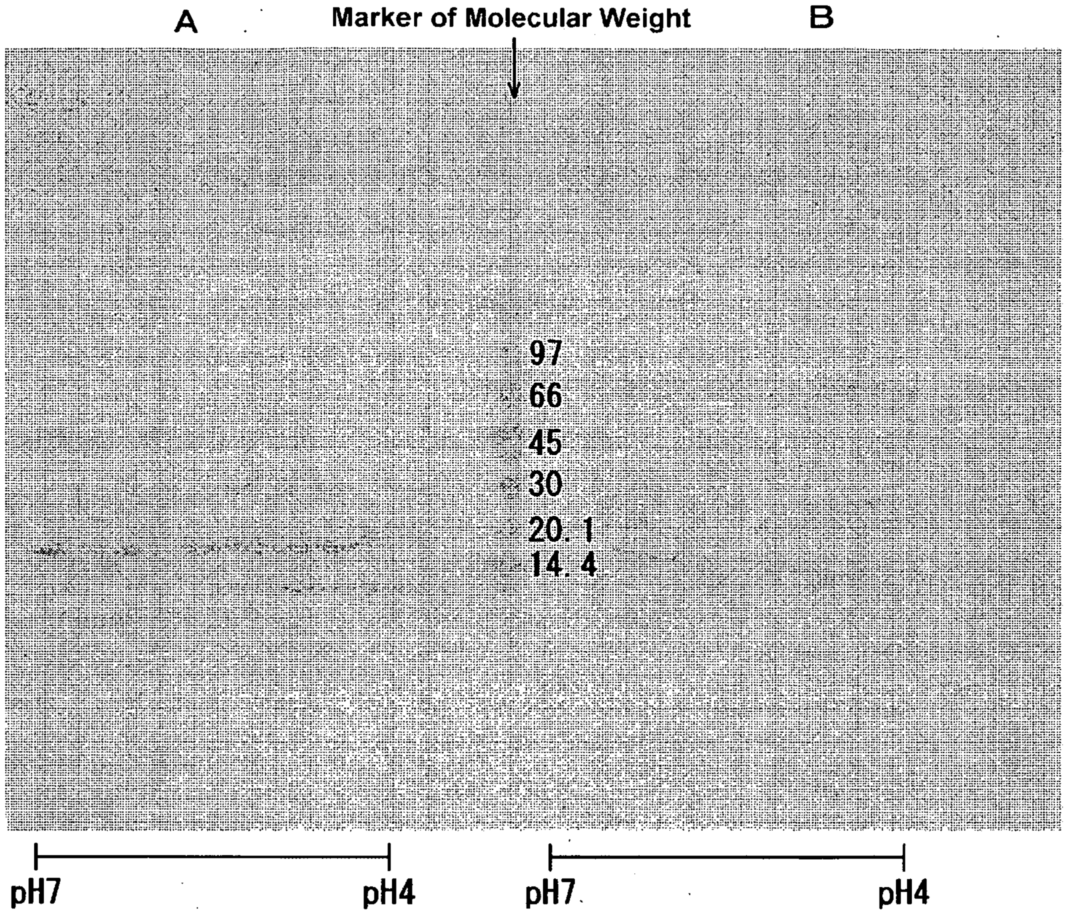
A
Marker of Molecular Weight
B
97
66
45
30
20. 1
14. 4
pH7
pH4
pH7
pH4

# Fig. 3

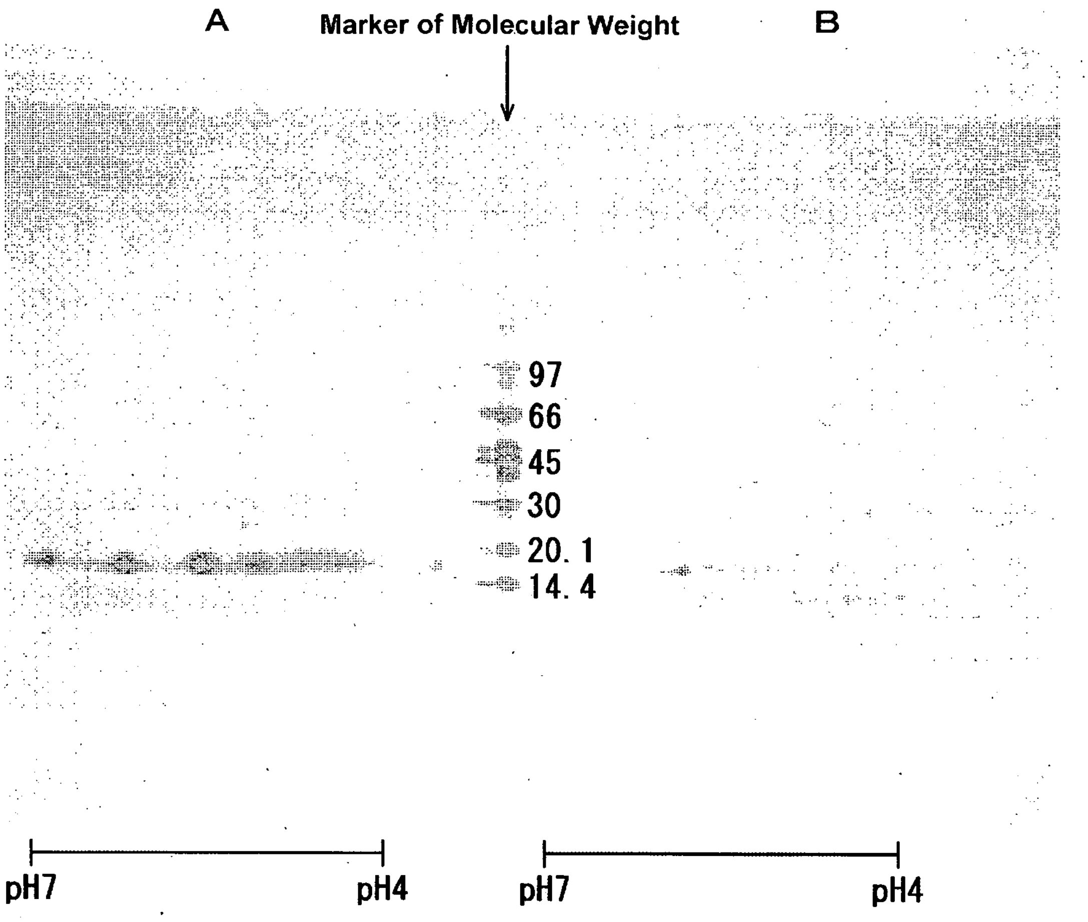
A
Marker of Molecular Weight
B
97
66
45
30
20.1
14.4
pH7
pH4
pH7
pH4

Fig. 4

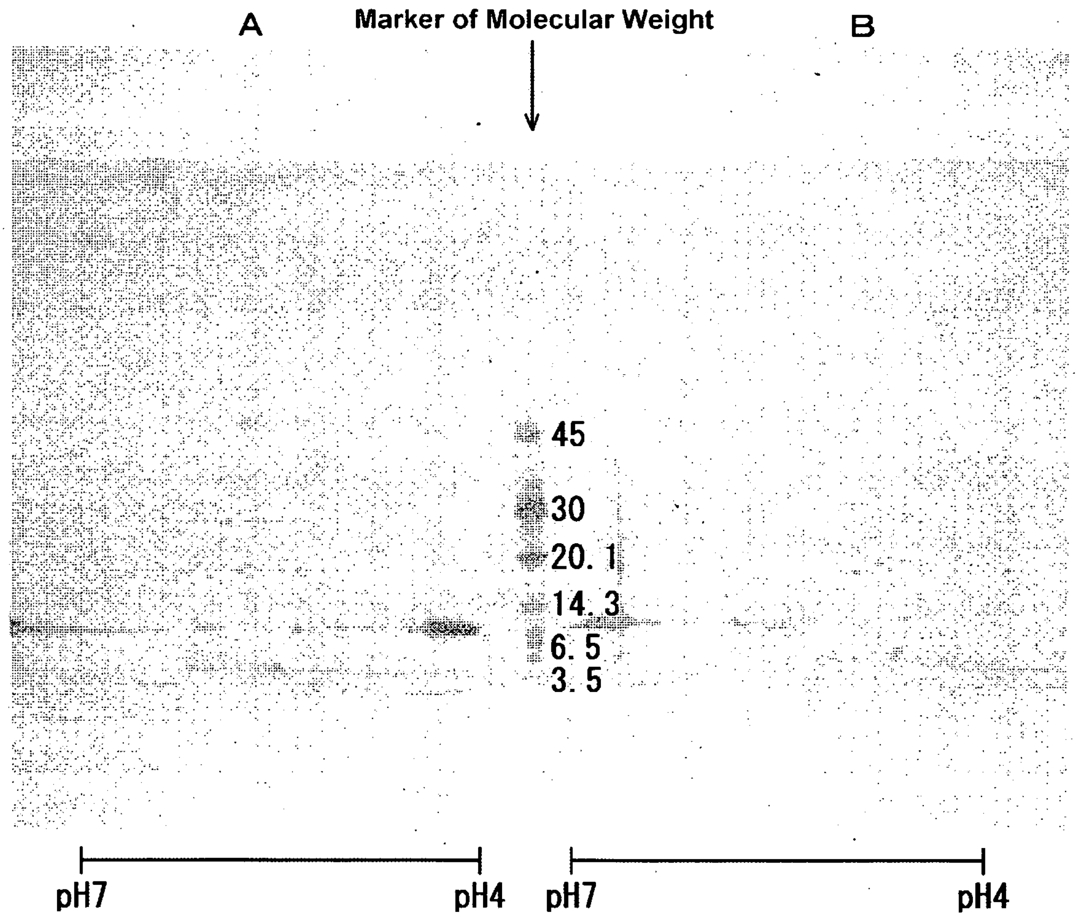
A
Marker of Molecular Weight
B
45
30
20. 1
14. 3
6. 5
3. 5
pH7
pH4
pH7
pH4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000005531 A
- JP 2001214367 A
- JP 56063355 A
- JP 61258806 A


### Non-patent literature cited in the description


- **Hirofusa Shirai ; Nagao Kobayashi.** phthalocyaninechemistry and function. Industrial Publishing & Consulting, Inc, 28 February 1997